# EUROPÄISCHE PATENTSCHRIFT

(11) **EP 0 975 671 B1**
(45) Veröffentlichungstag und Bekanntmachung des Hinweises auf die Patenterteilung: **24.09.2003**
(21) Anmeldenummer: 98901239.8
(22) Anmeldetag: 30.01.1998
(51) Int. Cl.: C07K 14/755

(54) **VERFAHREN ZUR CHROMATOGRAPHISCHEN REINIGUNG BZW. FRAKTIONIERUNG VON VON WILLEBRAND-FAKTOR AUS EINEM vWF-HÄLTIGEN AUSGANGSMATERIAL**
METHOD OF CHROMATOGRAPHICALLY PURIFYING OR FRACTIONATING VON WILLEBRAND FACTOR FROM A VWF-CONTAINING STARTER MATERIAL
PROCEDE DE PURIFICATION OU DE FRACTIONNEMENT CHROMATOGRAPHIQUE DU FACTEUR WILLEBRAND CONTENU DANS UN MATERIAU DE DEPART

(30) Priorität: 04.02.1997 AT 17697
(43) Veröffentlichungstag der Anmeldung: 02.02.2000
(73) Patentinhaber: Baxter Aktiengesellschaft, 1221 Wien (AT)
(72) Erfinder: SIEKMANN, Jürgen, A-1210 Wien (AT); TURECEK, Peter, A-3400 Klosterneuburg (AT); SCHWARZ, Hans-Peter, A-1180 Wien (AT); EIBL, Johann, A-1180 Wien (AT); FISCHER, Bernhard, A-1160 Wien (AT); MITTERER, Artur, A-2304 Mannsdorf 116 (AT); DORNER, Friedrich, A-1230 Wien (AT)
(74) Vertreter: Alge, Daniel, Mag. Dr. rer.nat.
(86) Internationale Anmeldenummer: AT9800020
(87) Internationale Veröffentlichungsnummer: WO98033820

(56) Entgegenhaltungen:
- EP-A- 0 023 607
- EP-A- 0 503 991
- S.A.SANTORO ET AL.: "Adsorption of von Willebrand Factor by Fibrillar Collagen- Implications Concerning the Adhesion of Platelets to Collagen" COLLAGEN REL.RES., Bd. 2, 1982, Seiten 31-43, XP002068370 in der Anmeldung erwähnt
- J.E.BROWN ET AL.: "An Elisa Test for the Binding of von Willebrand Factor Antigen to Collagen" THROMB.RES., Bd. 43, 1986, Seiten 303-311, XP002068371 in der Anmeldung erwähnt

## Beschreibung

Die Erfindung betrifft ein Verfahren zur chromatographischen Reinigung bzw. Fraktionierung von von Willebrand-Faktor (vWF) aus einem vWF-hältigen Ausgangsmaterial.

Dem vWF kommt eine besondere Bedeutung in der physiologischen Hämostase zu. Neben seiner Funktion als Carrier-Protein für Faktor VIII ist dieses Plasmaprotein vor allem für die Adhäsion von Thrombozyten an das geschädigte Endothel/Subendothel sowie für die Aggregation der Thrombozyten unter Scherstreßbedingungen notwendig. Im ersten Schritt der primären Hämostase, der Adhäsion, fungiert der vWF als ein Bindeglied zwischen spezifischen Rezeptoren der Thrombozytenoberfläche, wie gpIb, gpIIb/IIIa-Komplex, und Komponenten des Endothels, beispielsweise Kollagen.

Gemäß der EP 0 503 991 A wird vWF aus vorgereinigtem Plasma durch die Kombination von drei chromatographischen Reinigungsschritten in gereinigter Form erhalten. Diese chromatographischen Reinigungsschritte umfassen eine zweifache Chromatographie an einem Ionenaustauscher und als dritten Schritt eine affinitätschromatographische Reinigung an Gelatinesepharose. Es zeigte sich, daß bei der Gelatinesepharosechromatographie kontaminierende Proteine, wie beispielsweise Fibronektin, gebunden werden und der vWF im Eluat erhalten werden kann.

Die Reinigung von vWF durch eine Fraktogel-EMD-TMAE-Chromatographie mit anschließender Chromatographie an Heparin-Fraktogel-EMD wurde von Fischer et al. (Thromb. Res. 84 (1) (1996), 55-66) beschrieben.

Kollagen ist ein physiologischer Bindungspartner von vWF. Studien von Kessler et al. (Blood 63 (6) (1984), 1291-1298) ergaben, daß der Komplex von vWF und Faktor VIII an Kollagen-Fibrillen, nicht jedoch an denaturiertem Kollagen bindet. Dieses Ergebnis wurde oftmals bestätigt, beispielsweise durch Santoro et al. (Collagen Rel. Res. 2 (1982), 31-43), welche feststellten, daß nur natives Kollagen, nicht jedoch ein denaturiertes Kolla gen einen Bindungspartner für vWF darstellt (siehe auch Santoro, Thromb. Res. 21 (1981), 689-693, und BBA 756 (1983), 123-126; bestätigt auch durch die EP 0 503 991 A, da vWF die Gelatinesepharosesäule ungehindert passierte). Es zeigte sich, daß die hochmolekularen Formen von vWF durch limitierende niedrige Kollagenkonzentrationen gebunden wurden. Hochund mittelmolekulare Formen des vWF wurden auch durch nicht-limitierende hohe Kollagenkonzentrationen gebunden, wogegen die niedermolekulare vWF-Fraktion sogar bei hohen Kollagenkonzentrationen nicht gebunden wurde (Santoro (1983)).

In der EP 0 023 607 wird beschrieben, dass Faktor VIII:C, Faktor VIII R:AG und vWF aus Plasma durch Versetzen mit einer Kollagenlösung entfernt werden können.

Dies war auch der Grund dafür, daß bei Testmethoden, welche auf der vWF-Kollagenbindung basierten, stets natives Kollagen verwendet wurde und dessen Nativität auch im Test beibehalten wurde, indem denaturierende Bedingungen oder starke bzw. kovalente Bindungen des Kollagens an einen festen Träger bei diesen Bindungsassays nicht beschrieben wurden (Brown et al., Thromb. Res. 43 (1985), 303-311).

Obgleich die Bindung von Kollagen an vWF ausführlich beschrieben wurde und sogar vorgeschlagen wurde, diese Bindung von vWF an natives Kollagen bei einer vWF-Reinigungsmethode einzubeziehen (Santoro et al. (1982)), konnte bisher kein geeignetes präparatives Verfahren auf Basis der Kollagen-vWF-Bindung zur Verfügung gestellt werden. Dies lag einerseits daran, daß auf Kollagen basierende Chromatographiematerialien (wie z.B. Gelatinesepharose) sich als ungeeignet zur vWF-Bindung herausgestellt haben und andererseits daran, daß Kollagen-Fibrillen für die präparative Gewinnung des vWF nicht geeignet sind (siehe Santoro et al., 1982).

Aufgabe der vorliegenden Erfindung ist es, trotz dieser Bedenken ein Verfahren zum Reinigen bzw. Fraktionieren von vWF bereitzu stellen, welches auf der physiologischen Bindung von vWF zu Kollagen basiert, jedoch trotzdem industriell anwendbar ist und einen hohen Anteil an physiologisch aktivem vWF ermöglicht.

Diese Aufgabe wird erfindungsgemäß durch ein Verfahren der eingangs geschilderten Art gelöst, welches die folgenden Schritte aufweist:
- Adsorbieren des vWF aus dem Ausgangsmaterial an einem Träger immobilisierten Kollagen, ausgenommen Gelatinesepharose, wobei das Kollagen ausgewählt ist aus nativem Kollagen, einem Kollagenderivat, insbesondere enzymatisch prozessiertes Kollagen, oder einem Kollagenfragment, wobei die Bindungseigenschaft des Kollagens zu vWF nicht beeinträchtigt wird,
- Abtrennen des nicht adsorbierten Anteils und gegebenenfalls Waschen des Trägers,
- Eluieren des vWF vom immobilisierten Kollagen und
- Gewinnen des gereinigten vWF.

Das erfindungsgemäß eingesetzte Kollagen ist ein avides Kollagen mit einer hohen Bindungskapazität gegenüber vWF. Es ist insbesondere durch eine Vielzahl von zugänglichen Bindungsstellen für den vWF gekennzeichnet. Die Kriterien zur Auswahl eines geeigneten Kollagens sind vielfältig. Einerseits kann von einem Material ausgegangen werden, welches bekanntermaßen schon geeignete Bindungsfähigkeiten besitzt und gegebenenfalls zur Erhöhung der Stabilität prozessiert wird. Die Prozessierung kann aber auch mit dem Ziel der Vergrößerung bzw. Homogenität der Oberfläche des immobilisierten Kollagens erfolgen, was Voraussetzung für das reproduzierbare Ausmaß der Adsorption von vWF ist. Überraschenderweise konnte die Avidität von Kollagen im Gegensatz zu den Untersuchungen im Stand der Technik auch bei immobilisiertem Kollagen genutzt werden, obwohl es mit der Immobilisierung zwangsläufig zu einer Denaturierung des Kollagenmoleküls kommt, insbesondere, wenn die Immobilisierung durch kovalente Bindung und durch starke Wechselwirkungskräfte erfolgt.

Die Immobilisierung des Kollagens an den festen Träger kann direkt oder indirekt, beispielsweise über Abstandshalter (sogenannte Spacer) erfolgen. Dadurch werden Probleme, die sich aufgrund sterischer Hinderungen von Träger und zu immobilisierendem Liganden ergeben können, weitgehend vermieden. Beispielsweise werden als Spacer homo- oder heterobifunktionelle "cross-linker"-Substanzen, wie Hexamethylendiamin, 3,3'-Diaminopropyl amin, 6-Aminohexansäure oder das heterobifunktionelle SPDP-Reagens der Firma Pharmacia (Uppsala, Schweden), verwendet.

In einer bevorzugten Ausführungsform wird das Trägermaterial vorher mittels gängiger Methoden aktiviert, beispielsweise durch Behandeln mit Carbodiimid oder Maleinsäure-Anhydrid.

Es stellte sich überraschenderweise heraus, daß vWF trotz der Bindung von Kollagen zum festen Träger durch starke non-kovalente Wechselwirkung oder durch kovalente Bindung eine ausreichende Bindung ermöglichte. Dies war insbesondere in Kenntnis der Ergebnisse von Santoro et al. und auch der EP 0 503 991 A1 überraschend, da in diesen Untersuchungen übereinstimmend herausgefunden worden ist, daß vWF an denaturiertes Kollagen nicht gebunden wurde.

Das Vorliegen dieser starken, nicht-kovalenten oder kovalenten Bindung kann beispielsweise mittels des nachfolgend beschriebenen Tests bestätigt werden: Das an einem festen Träger immobilisierte Kollagen, an dem gegebenenfalls vWF adsorbiert ist, wird hierzu mit 0,4 mol/l NaOH für 15 Minuten bei Raumtemperatur behandelt. Der Kollagen- bzw. vWF-Gehalt des mit NaOH behandelten Materials sowie des unbehandelten Materials wird bestimmt und aus der Differenz der beiden Bestimmungen der Verlust an gebundenem Kollagen und gegebenenfalls vWF bzw. die Abnahme der Bindungskapazität für vWF ermittelt. Ein erfindungsgemäß verwendetes immobilisiertes Kollagen liegt dann vor, wenn der Verlust an Kollagen bzw. an vWF nach Behandlung mit NaOH nicht mehr als 20 %, vorzugsweise nicht mehr als 10 %, beträgt.

Es zeigte sich, daß mit dem erfindungsgemäßen Verfahren nicht nur vWF in hoher Ausbeute gewonnen werden kann, sondern auch vWF-Fraktionen von sehr gut definierten Bindungsaktivitäten. Demgemäß wird vWF vorzugsweise in mehr als einer Fraktion gewonnen, welche Fraktionen vWF mit unterschiedlichen Aktivitäten, insbesondere mit verschiedenen Kollagenbindungsaktivitäten, enthalten. Überraschenderweise zeigte sich, daß der vWF bei dem erfindungsgemäßen Verfahren nicht ausschließlich nach Molekulargewicht fraktioniert wird. Üblicherweise sind aber hochaktive vWF-Fraktionen allerdings solche, die im wesentlichen vWF mit einem hochmolekularen Anteil, insbesondere hochmolekulare vWF-Multimere mit hoher struktureller Integrität, enthalten.

Diese unterschiedlichen Fraktionen können beispielsweise durch Erhöhung der Ionenstärke bzw. der Elutionsstärke (pH, Chaotrope) bei der Elution gewonnen werden. Vorzugsweise wird der vWF in einer ersten Fraktion und zumindest in einer zweiten Fraktion gewonnen, wobei die Elution der zweiten Fraktion vorteilhafterweise mit einer Lösung, welche eine gegenüber der Elutionslösung für die erste Fraktion um mindestens 20 % erhöhte Ionenstärke aufweist, durchgeführt.

Die Elution wird beispielsweise als Gradientenelution durchgeführt.

Vorzugsweise wird eine erste Fraktion erhalten, in der der vWF eine Kollagenbindungsaktivität von weniger als 0,4 E vWF:CB/E vWF:Ag aufweist bzw. eine zweite Fraktion, in der der vWF eine Kollagenbindungsaktivität von mehr als 0,6 E vWF:CB/E vWF:Ag aufweist.

Es hat sich gezeigt, daß die Elution des vWF mit dem erfindungsgemäßen Verfahren mit einem Puffer mit einer Ionenstärke entsprechend einer Natriumchloridkonzentration von mindestens 150 mM, insbesondere entsprechend einer NaCl-Konzentration im Bereich von 150 mM bis 400 mM, vorgenommen werden kann.

Gemäß einer weiteren bevorzugten Verfahrensvariante wird die Elution eines vWF mit einem Puffer mit einer Ionenstärke entsprechend einer NaCl-Konzentration von weniger als 200 mM, insbesondere entsprechend einer NaCl-Konzentration im Bereich von 50 mM bis 150 mM, vorzugsweise in einem Medium mit physiologischer Ionenstärke, vorgenommen.

Erfindungsgemäß kann durch Elution unter physiologischen Bedingungen die native Struktur von vWF in einem viel höheren Maße beibehalten werden, was nicht nur eine einheitlichere vWF-Präparation ermöglicht, sondern auch ein Minimum an Nebenreaktionen, beispielsweise durch Neoantigene, bei der Verabreichung eines derartigen vWF-Präparates an den Menschen gewährleistet.

In einer weiteren bevorzugten Ausführungsform sind im Puffer Ca²⁺-Ionen enthalten. Beispielsweise ist der Gehalt an Ca²⁺-Ionen im Puffer geringer als 10 mM, vorzugsweise geringer als 5 mM, sodaß Faktor VIII-vWF-Komplex an immobilisiertem Kollagen adsorbiert wird. Gegebenenfalls kann der Ca²⁺-Ionen-Gehalt bei der Elution auf 150 - 200 mM erhöht werden, sodaß der Komplex gespalten wird und vWF aus dem Komplex gewonnen wird.

Der pH-Wert des Elutionspuffers beträgt vorzugsweise 5 bis 9, jedoch hat sich auch ein Puffer mit einem pH von 3 bis 4, insbesondere bei einer Ionenstärke von 150 mM NaCl, als vorteilhaft herausgestellt. Weiters umfaßt der Elutionspuffer bevorzugterweise chaotrope Salze, insbesondere Ammonium- und/ oder Kaliumthiocyanat, wobei eine Konzentration von 1 bis 3 M besonders bevorzugt wird.

Da Fibronektin ebenfalls eine Affinität zu Kollagen aufweist, jedoch (siehe EP 0 503 991 A1) auch zu denaturiertem Kollagen, ist es möglich, gemäß dem erfindungsgemäßen Verfahren in einer weiteren Fraktion auch Fibronektin präparativ zu gewinnen. Das erfindungsgemäße Verfahren eignet sich also auch zur chromatographischen Reinigung von Fibronektin.

Als Ausgangsmaterial für das erfindungsgemäße Verfahren eignet sich jede Lösung, welche vWF enthält. Insbesondere ist das vWF-hältige Ausgangsmaterial humanes Plasma, eine Plasmafraktion oder ein Zellkulturüberstand. Vorzugsweise wird aber von einem vWF-Konzentrat oder einem kommerziell erhältlichen Faktor VIII/vWF-Komplex-hältigen Präparat ausgegangen.

Vorteilhafterweise wird vor dem erfindungsgemäßen Adsorptionsschritt an immobilisiertem Kollagen eine Vorreinigung des vWF-hältigen Ausgangsmaterials durchgeführt. Hiefür sind die bereits im Stand der Technik beschriebenen Verfahren der Ionenaustauschchromatographie, Gelpermeationschromatographie und/oder eine Fällung, vorzugsweise eine Fällung mit Glycin oder Ammoniumsulfat, besonders geeignet.

In einer bevorzugten Ausführungsform ist das erfindungsgemäße Verfahren als terminales Reinigungsverfahren geeignet. Die Formulierung als pharmazeutisches Präparat vereinfacht sich damit, da aufgrund der physiologischen Ionenstärke (0,13-0,16 M) bzw. der physiologischen Osmolalität (240-350, vorzugsweise 275 mOs/kg) des verwendeten Mediums für die Elution keine weiteren Reinigungsschritte notwendig sind. Insbesondere bietet dieses Verfahren auch einen Vorteil gegenüber der Immunaffinitätschromatographie, da hier wegen des möglichen Ausblutens von Immunglobulin weitere Reinigungsschritte unbedingt erforderlich sind.

Es zeigte sich, daß erfindungsgemäß eine ganze Reihe von Kollagentypen verwendet werden können, insbesondere Kollagen Typ I oder Typ III (siehe z.B. Hörmann H., Hämostaseologie 1990, 10; 138-46). Gerade die kommerziell erhältliche Gelatinesepharose erwies sich aber (vgl. EP 0 503 991 A1) als ungeeignet für das erfindungsgemäße Verfahren, wenngleich andere, kommerziell erhältliche Kollagen-Sepharosen durchaus im erfindungsgemäßen Verfahren angewendet werden können, z.B. Kollagen-Agarose von Sigma.

Als Kollagen wird entweder Kollagen humanen Ursprungs oder ein Kollagen, welches vom menschlichen Kollagen möglichst weit entfernt ist, also beispielsweise vom Schwein, Rind, Ratte oder Pferd stammt, verwendet.

Der Kollagenträger ist vorzugsweise ein Chromatographiematerial, an das das Kollagen z.B. kovalent gebunden ist, damit das erfindungsgemäße Verfahren insbesondere im industriellen Maßstab eingesetzt werden kann.

Bevorzugterweise weist das erfindungsgemäß eingesetzte Kollagen eine Modifikation auf, durch welche eine stabile Immobilisierung an den festen Träger ausgebildet wird. Selbstverständlich sollte dabei die Bindungseigenschaft zum zu reinigenden Protein nicht beeinträchtigt werden. Diese Modifikation kann beispielsweise darin bestehen, daß im Kollagenmolekül reaktive Aldehydgruppen vorgesehen werden.

Überraschenderweise erwies sich auch prozessiertes Kollagen als geeignet für das vorliegende Verfahren, wenn durch die Prozessierung die Avidität des Kollagen im wesentlichen erhalten bleibt. Das avide Kollagen, welches erfindungsgemäß eingesetzt werden kann, ist vorzugsweise aus niedermolekularem Kollagen, aus proteolytisch prozessiertem, z.B. Pepsin verdautem, desintegriertem oder homogenisiertem Kollagen abgeleitet. Das Kollagen kann auch chemisch behandelt bzw. modifiziert (derivatisiert) sein; auch Kollagenfragmente können erfindungsgemäß eingesetzt werden. Vorteilhafterweise wird natives Kollagen oder ein Kollagenderivat, beispielsweise ein Peptid abgeleitet von Kollagen, eingesetzt.

In einer bevorzugten Ausführungsform ist der vWF und/oder das Kollagen zur Inaktivierung und/oder Abreicherung von Pathogenen, insbesondere von Viren, behandelt.

Zur Inaktivierung von Viren sind eine Reihe von physikalischen, chemischen oder chemisch/physikalischen Methoden bekannt, die beispielsweise eine Hitzebehandlung, z.B. gemäß der EP-0 159 311 A oder der EP-0 637 451 A, eine Hydrolasebehandlung gemäß der EP-0 247 998 A, eine Strahlenbehandlung oder eine Behandlung mit organischen Lösungsmitteln und/oder Tensiden, z.B. gemäß der EP-0 131 740 A, umfassen. Weitere geeignete Virusinaktivierungsschritte bei der Herstellung der erfindungsgemäßen Präparate sind in der EP-0 506 651 A oder in der WO-94/13329-A beschrieben.

Ein Verfahren zur Inaktivierung bzw. Abreicherung von Viren kann - auch einen Filtrationsschritt, beispielsweise eine Tiefen-, Ultra- oder Nanofiltration, beinhalten. Neben dem vWF kann auch das Kollagen alleine oder gemeinsam mit diesem entsprechend behandelt werden, beispielsweise kann Kollagen hitzebehandelt werden.

Wird das vWF-hältige Ausgangsmaterials beispielsweise durch Zugabe von Detergens behandelt, so kann dieses Detergens enthaltende Ausgangsmaterial auch direkt am immobilisierten Kollagen adsorbiert werden. Dadurch kann die Bindungskapazität des Kollagens für vWF sogar erhöht werden.

Gemäß einem weiteren Aspekt betrifft die vorliegende Erfindung eine Präparation zur Herstellung eines gereinigten vWF, welche biologisch aktiven vWF, der an immobilisiertes Kollagen gebunden ist, umfaßt als auch eine pharmazeutische Präparation umfassend biologisch aktiven vWF, der stabil an Kollagen gebunden ist.

Eine besondere Ausführungsform der erfindungsgemäßen Präparationen ist dadurch gekennzeichnet, daß der vWF eine primäre hämostatische Aktivität, ausgedrückt durch eine Kollagenbindungsaktivität von wenigstens 0,9 E vWF:CB/E vWF-Antigen, vorzugsweise mindestens 1 E vWF:CB/E vWF-Antigen, und eine Reinheit von wenigstens 70 E vWF-Antigen/mg Protein, vorzugsweise mindestens 80 E vWF-Antigen/mg Protein, aufweist.

Derartige vWF-Präparationen können vorteilhafterweise mit dem erfindungsgemäßen Verfahren hergestellt werden, sind jedoch nicht auf dieses beschränkt.

Vorteilhafterweise besitzt der vWF in den erfindungsgemäßen Präparationen eine primäre hämostatische Aktivität, ausgedrückt in Ristocetin-Cofaktor-Einheiten (E vWF:RCo) pro Einheit vWF:Ag von wenigstens 0,3, vorzugsweise zumindest 0,4, insbesondere von mindestens 0,6.

Weiters sollte der vWF gemäß den erfindungsgemäßen Präparationen eine Faktor VIII-Bindungskapazität aufweisen.

Gemäß weiteren vorteilhaften Ausführungsformen enthalten die erfindungsgemäßen Präparationen eine vWF-Präparation, welche frei von plasmatischen Proteinen ist, bzw. einen vWF, welcher als Faktor VIII- vWF-Komplex vorliegt.

Vorteilhafterweise ist das Kollagen in den erfindungsgemäßen Präparationen an einen festen Träger immobilisiert, welcher aus der Gruppe Kollagenpartikel, synthetisches Trägermaterial, Material auf Kohlehydratbasis, Phospholipid und Liposom ausgewählt sein kann. Auch anorganische Materialien, wie beispielsweise die als Adjuvantien gängigen Metallhydroxid-Verbindungen, kommen in Frage. Die Kollagenpartikel können globulärer Natur sein, aber auch Ketten, Fibrillen oder Fasern. Als fester Träger kann auch Kollagen per se verwendet werden, wenn es derart prozessiert ist - wie bereits an früherer Stelle angemerkt - daß es als Träger geeignet ist.

Geeignete medizinische Verwendungsmöglichkeiten umfassen die Verwendung der erfindungsgemäßen Präparation zur Herstellung einer Präparation zur Behandlung der Hämophilie A sowie von Erkrankungen, die mit einem angeborenen oder erworbenen Mangel an vWF einhergehen. Diese Krankheiten sind unter dem Begriff der funktionellen Störungen des vWF zu subsumieren.

Durch das zur Verfügungstellen von vWF in an Kollagen gebundener Form wird ein physiologisches Arzneimittel zur Verfügung gestellt, welches in vivo, beispielsweise einen an Thrombozyten gebundenen vWF imitieren kann. Besondere Anwendungsmöglichkeiten umfassen daher auch alle jene physiologischen Funktionen, bei welchen die Bindung von vWF an Thrombozytenoberflächen gestört ist bzw. bei welchen eine zusätzliche derartige Aktivität benötigt wird.

Die pharmazeutische Präparation wird vor allem, wenn sie über eine bestimmte Zeit gelagert werden soll, vorteilhafterweise in lyophilisierter Form zur Verfügung gestellt.

Die pharmazeutische Präparation liegt vorzugsweise in einer für die parenterale (z.B. i.v., i.m. oder s.c.), mukosale (z.B. oral) oder topische (z.B. als Salbe) Anwendung geeigneten Form vor. Beispielsweise wird sie als Suspension oder als Lösung zur Verfügung gestellt.

Die Formulierung der erfindungsgemäß angewandten Präparation kann in an sich bekannter und üblicher Weise erfolgen, z.B. mit Hilfe von Salzen und gegebenenfalls Aminosäuren, aber auch in Gegenwart von Tensiden vorgenommen werden. Vorzugsweise werden Salze, wie z.B. Natriumchlorid oder Kalziumchlorid, verwendet und ein pH im Bereich von 6 bis 8 gewählt. Als Aminosäuren sind Glycin oder Lysin bevorzugt. Ebenso kann ein pharmazeutisch akzeptabler Puffer gewählt werden.

Die zu verabreichende Dosis hängt insbesondere von der zu behandelnden Krankheit ab und entspricht im wesentlichen den für Faktor VIII-vWF-Komplex bzw. vWF-Präparationen verabreichten Mengen.

Eine vorteilhafte Depot- bzw. Langzeitwirkung der erfindungsgemäßen pharmazeutischen Präparation ist insbesondere aufgrund des vorhandenen Kollagens gegeben.

Zweckmäßigerweise sind die erfindungsgemäßen Präparationen auch zur Inaktivierung gegebenenfalls vorhandener Pathogene, insbesondere Viren, behandelt.

Die vorliegende Erfindung wird durch die nachfolgenden Beispiele sowie die Zeichnungsfiguren, auf die sie jedoch nicht beschränkt sein soll, näher erläutert.

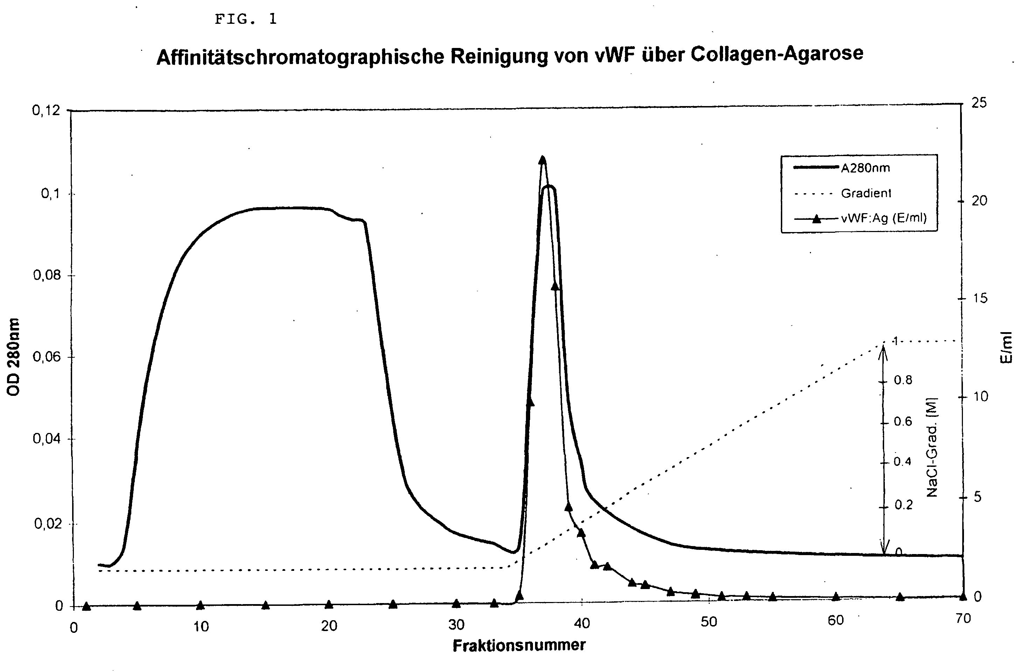

Fig. 1 zeigt das Chromatogramm für die affinitätschromatographische Reinigung von vWF über Kollagen-Agarose.

### Beispiel 1: Reinigung des vWF

Ein Faktor VIII-vWF-Komplex-Konzentrat, hergestellt gemäß der AT 391 808, enthaltend von Willebrand-Faktor in einer Konzentration von 260 E/ml vWF:Ag und einer spezifischen Aktivität von 13,5 E vWF:Ag/mg Protein, wurde mit 20 mmol/l Tris-Puffer, pH 7,4 (= Puffer A) auf eine vWF-Konzentration von 6 E/ml vWF:Ag verdünnt. 20 ml dieser Lösung wurden auf eine Affinitätssäule (1,0 x 4,0 cm), gefüllt mit Kollagen-Agarose (Sigma C-0286), aufgetragen. Nach Spülen der Säule mit 10 ml Puffer A wurde mit NaCl eluiert. Dazu wurde ein linearer Gradient von 0 bis 1 mol/l NaCl in Puffer A über 30 ml angelegt. Danach wurde mit 10 ml Puffer B (20 mmol/l Tris, 1 mol/l NaCl, pH 7,4) nachgewaschen. Die gesamte Chromatographie wurde bei einer Flußrate von 1 ml/min durchgeführt. Es wurden 1 ml-Fraktionen gesammelt und deren optische Dichte bei 280 nm bestimmt. Von allen Fraktionen wurde der Gehalt an vWF-Antigen mit Hilfe eines ELlSA's (ASSERACHROM vWF, Fa. Boehringer, Mannheim) bestimmt. Die Messungen zeigten, daß im Durchlauf kein vWF im Antigen-Test nachgewiesen werden konnte und daß eine vWF-Antigenmenge von 40 E pro ml Kollagen-Agarose gebunden wurde. Der von Willebrand-Faktor eluierte bei einer Kochsalz-Konzentration von 100 mmol/l. Das Elutionsdiagramm wird in Fig. 1 dargestellt: Nach Bestimmung des Proteingehaltes mit Hilfe des Bradford-Assays (Anal.Biochem. 72, 248-254, 1976) ergab sich im Elutionsmaximum eine Reinheit des vWF von 85 E vWF:Ag/mg Protein. Für die Aktivität des vWF wurden Werte von 0,47 E vWF:RCo/E vWF:Ag sowie 0,45 E vWF:CB/E vWF:Ag ermittelt.

### Beispiel 2: Fraktionierung des vWF

Ein gemäß der EP 0 567 448 hochgereinigtes vWF-Konzentrat, enthaltend von Willebrand-Faktor in einer Konzentration von 105 E/ml vWF:Ag und einer spezifischen Aktivität von 60 E vWF:Ag/mg Protein, wurde mit 20mmol/l Tris-Puffer, pH 7,4 (= Puffer A) auf eine vWF-Konzentration von 4 E/ml vWF:Ag verdünnt. 20 ml dieser Lösung wurden auf eine Affinitätssäule (1,0 x 4,0 cm), gefüllt mit Kollagen-Agarose (Sigma 0-0286), aufgetragen. Nach Spülen der Säule mit 10 ml Puffer A wurde mit einem NaCl-Stufengradienten eluiert. Dazu wurde die Säule mit jeweils 10 ml Puffer B (0,15 mol/l NaCl in Puffer A), Puffer C (0,3 mol/l NaCl in Puffer A), Puffer D (0,5 mol/l NaCl in Puffer A) und Puffer E (1 mol/l NaCl in Puffer A) eluiert. Puffer E dient der Regenerierung der Säule. Die gesamte Chromatographie wurde bei einer Flußrate von 1 ml/min durchgeführt. Es wurden 1 ml-Fraktionen gesammelt und deren optische Dichte bei 280 nm bestimmt. Von allen Fraktionen wurde der Gehalt an vWF-Ahtigen mit Hilfe eines ELlSA's (ASSERACHROM vWF, Fa. Boehringer, Mannheim) bestimmt. Die Messungen zeigten, daß eine Antigenmenge von 23 E pro ml Kollagen-Agarose gebunden wurde und daß eine Antigenmenge von insgesamt 3 E im Durchlauf eluierte. Der von Willebrand-Faktor eluierte mit den Puffern B, C und D von der Säule und wies eine Reinheit von 75 E vWF:Antigen/mg Protein auf. In den verschiedenen Eluaten wurden die folgenden Aktivitäten ermittelt:

| E vWF:RCo/E vWF:Ag | | Aktivität E VWF:CB/E vWF:Ag |
|---|---|---|
| Eluat Puffer B | 0,41 | 0,46 |
| Eluat Puffer C | 0,68 | 0,70 |
| Eluat Puffer D | 0,91 | 0,87 |

### Beispiel 3: Reinigung des Faktor VIII-vWF-Komplexes

Zur Isolierung eines Faktor VIII-vWF-Komplexes wurde ein Kryopräzipitat, hergestellt gemäß Pool et al. (N.Engl.J.Med. 273, 1443-1447, 1965), in 20 mmol/l Tris-Puffer, pH 7,4 aufgelöst. 20 ml einer Lösung enthaltend 6 E vWF:Ag/ml und 5,5 E/ml Faktor VIII:c (IMMUNOCHROM F VIII:c-Test, Fa. lmmuno) auf eine Säule gefüllt mit Kollagen-Agarose aufgetragen. Die Affinitätschromatographie wurde analog zu Beispiel 1 durchgeführt. Von jeder Fraktion wurde der Gehalt an von Willebrand-Faktor-Antigen sowie an Faktor VIII:c bestimmt. Der von Willebrand-Faktor wurde im Komplex mit Faktor VIII:c zusammen bei einer Kochsalzkonzentration von 100 mmol/l von der Säule eluiert.

### Beispiel 4: Herstellung eines hochgereinigten vWF-Konzentrats

Zur Herstellung eines hochgereinigten von Willebrand-Faktor-Konzentrats für den pharmazeutischen Gebrauch wurde entsprechend Beispiel 1 eine affinitätschromatographische Reinigung durchgeführt. Das Auftragen der Proben auf die Affinitätssäule erfolgte in 20 mmol/l Natriumcitrat-Puffer, pH 7,3. Die Fraktionen mit einer spezifischen Aktivität von mehr als 70 E vWF:Ag/mg Protein wurden gesammelt, durch Zusatz von 5 g Glycin und 5 g L-Lysin pro Liter stabilisiert, ein pH-Wert von 7,0 mit 0,1 mol/l NaOH eingestellt und anschließend gefriergetrocknet. Nach Rekonstitution mit H₂O wurde für diese Präparation eine Osmolalität (Pharmak. Europ., 2nd Ed. V.6.25) von 350 mOsmol/kg H₂O ermittelt.

### Beispiel 5: Immobilisierung von Kollagen (derzeit nach Ansicht der Anmelderin der beste Weg zur Ausführung der Erfindung)

Zur Herstellung eines Affinitätsharzes mit kovalent immobilisiertem Kollagen wurden 25 mg säurelösliches Typ-III Kollagen aus humaner Plazenta (Sigma C-4407) in 10 ml 0,1 M Essigsäure bei 4°C gelöst und über 6 h bei dieser Temperatur vorsichtig geschüttelt. Danach wurde durch ein Filter mit einem Porendurchmesser von 0,4 µm filtriert, das Filtrat zu einer Suspension von 5ml ECH-Sepharose® 4B (Pharmacia) gegeben und ein pH-Wert von 4,5 durch Zugabe von 0,1 mol/l NaOH eingestellt. Bei einer Temperatur von 4°C wurden über einen Zeitraum von 2 Stunden in Portionen zu 30 mg insgesamt 300 mg 1-Ethyl-3-(3-dimethylaminopropyl)carbodiimid-Hydrochlorid (EDC/Fa. Pierce) unter ständigem Rühren unter Verwendung eines mit einem Elektromotor betriebenen Rührstabes zugegeben. Anschließend wurde noch weitere 6 Stunden bei 4°C gerührt. Während der gesamten Reaktionszeit wurde der pH-Wert mit Hilfe einer pH-Elektrode kontrolliert und durch Zugabe von 0,1 mol/l NaOH in einem Bereich von 4,5 bis 5,0 gehalten. Das so hergestellte Affinitätsgel wurde zunächst mit 20 ml Natriumacetatpuffer (0,1 mol/l Acetat, 0,5 mol/l NaCl, pH 4,3) sowie mit 20 ml Trispuffer (0,1 mol/l Tris, 0,5 mol/l NaCl, pH 7,8) bei Raumtemperatur gewaschen und dieser Prozeß zweimal wiederholt. Nach Waschen des Gels mit H₂O sowie mit dem in der nachfolgenden Affinitätschromatographie verwendeten Puffersystem zur Bindung des vWF wurde das Gel wie unter den Beispielen 1 bis 4 beschrieben zur Aufreinigung von vWF eingesetzt.

### Beispiel 6: Reinigung von vWF mit modifiziertem Kollagen

### a) Auflösen von Kollagen

Humanes Kollagen Typ III wurde zu 5 mg/ml in 500 mM Essigsäure bei 4°C aufgelöst und anschließend mit Wasser auf 1 mg/ml verdünnt. Das gelöste Kollagen wurde bei -20°C gelagert.

### b) Chemische Modifikation von Kollagen

Zur chemischen Modifikation wurde das Kollagen von Beispiel 6 a) in 10 mM Na-Acetatpuffer, pH 4, zu 10 µg/ml verdünnt. Zu 10 ml dieser Lösung wurden 32 mg NaIO₄ zugegeben und die Reaktion für 0,5 Stunden bei Raumtemperatur durchgeführt. Diese Reaktion resultiert in einer chemischen Modifikation unter Herausbildung reaktiver Aldehydgruppen im Kollagenmolekül.

### c) Chemische Kopplung von modifiziertem Kollagen an einen festen Träger

Humanes Kollagen wurde entsprechend Beispiel 6 b) mit Na-Periodat aktiviert. Aktiviertes Kollagen wurde an einen Aminogruppen-haltigen Träger, Adipin-Hydrazid-Sepharose (Fa. Pharmacia), über den aktivierten Kohlehydratanteil des Kollagens und der Aminogruppe des Trägers kovalent gekoppelt.

### d) Reinigung von vWF aus einer vWF-hältigen Lösung

Zur Reinigung von vWF wurden 10 ml einer vWF-hältigen Lösung mit einer Ausgangsaktivität an vWF von 900 mU/ml vWF:RistoCoF an dem Träger bestehend aus Kollagen-Adipinsäure-Hydrazid-Sepharose in Kontakt gebracht und der vWF an den Träger gebunden. Nicht gebundene Proteine wurden vom Träger durch Waschen mit einem Puffer enthaltend 50 mM Tris/HCl, pH 7,4, 150 mM NaCl gewaschen. Gebundener vWF wurde vom Träger mit einem Puffer enthaltend 50 mM Tris/HCl, pH 7,4, 150 mM NaCl, 1,5 M KSCN eluiert. Das Eluat wurde auf vWF-Aktivität getestet. Im Eluat betrug die vWF-Aktivität 1500 mU/ml vWF:RistoCoF. Die gelelektrophoretische Analyse zeigt, daß das Eluat insbesondere hochmolekulare vWF-Multimere enthält.

## Patentansprüche

1. Verfahren zur chromatographischen Reinigung bzw. Fraktionietionierung von von Willebrand-Faktor (vWF) aus einem vWF-hältigen Ausgangsmaterial, umfassend die folgenden Schritte:
- Adsorbieren des vWF aus dem Ausgangsmaterial an einem Träger immobilisierten Kollagen, ausgenommen Gelatinesepharose, wobei das Kollagen ausgewählt ist aus nativem Kollagen, einem Kollagenderivat, insbesondere enzymatisch prozessiertes Kollagen, oder einem Kollagenfragment, wobei die Bindungseigenschaft des Kollagens zu vWF nicht beeinträchtigt wird,
- Abtrennen des nicht adsorbierten Anteils und gegebenenfalls Waschen des Trägers,
- Eluieren des vWF vom immobilisierten Kollagen und
- Gewinnen des gereinigten vWF.

2. Verfahren nach Anspruch 1, **dadurch gekennzeichnet, daß** der vWF in mehr als einer Fraktion gewonnen wird.

3. Verfahren nach Anspruch 1 oder 2, **dadurch gekennzeichnet, daß** vWF in einer ersten Fraktion und zumindest in einer zweiten Fraktion gewonnen wird, wobei die Elution der zweiten Fraktion mit einer Lösung, welche eine gegenüber der Elutionslösung für die erste Fraktion um mindestens 20% erhöhte Ionenstärke aufweist, durchgeführt wird.

4. Verfahren nach einem der Ansprüche 1 bis 3, **dadurch gekennzeichnet, daß** die Elution als Gradientenelution durchgeführt wird.

5. Verfahren nach einem der Ansprüche 1 bis 4, **dadurch gekennzeichnet, daß** eine erste Fraktion erhalten wird in der der vWF eine Kollagenbindungsaktivität von weniger als 0,4 E vWF:CB/E vWF:Ag aufweist bzw. eine zweite Fraktion in der der vWF eine Kollagenbindungsaktivität von mehr als 0,6 E vWF:CB/E vWF:Ag aufweist.

6. Verfahren nach einem der Ansprüche 1 bis 5, **dadurch gekennzeichnet, daß** die Elution der zweiten Fraktion mit einem Puffer mit einer Ionenstärke entsprechend einer NaCl-Konzentration von mindestens 150 mmol/l, insbesondere entsprechend einer NaCl-Konzentration im Bereich von 150 bis 400 mmol/l, vorgenommen wird.

7. Verfahren nach einem der Ansprüche 1 bis 6, **dadurch gekennzeichnet, daß** die Elution der ersten Fraktion mit einem Puffer mit einer Ionenstärke entsprechend einer NaCl-Konzentration von weniger als 200 mmol/l, insbesondere entsprechend einer NaCl-Konzentration im Bereich von 50 bis 150 mmol/l, vorzugsweise in einem Medium mit physiologischer Ionenstärke, vorgenommen wird.

8. Verfahren nach einem der Ansprüche 1 bis 7, **dadurch gekennzeichnet, daß** in einer weiteren Fraktion Fibronektin gewonnen wird.

9. Verfahren nach einem der Ansprüche 1 bis 8, **dadurch gekennzeichnet, daß** das vWF-hältige Ausgangsmaterial ein vWF-Konzentrat oder ein Faktor VIII-vWF-Komplex-hältiges Präparat ist.

10. Verfahren nach einem der Ansprüche 1 bis 9, **dadurch gekennzeichnet, daß** vor dem Adsorptionsschritt an immobilisiertem Kollagen eine Vorreinigung des vWF-hältigen Ausgangsmaterials, insbesondere eine Ionenaustauschchromatographie, eine Gelpermeationschromatographie und/oder eine Fällung, vorzugsweise eine Fällung mit Glycin oder Ammoniumsulfat, durchgeführt wird.

11. Verfahren nach einem der Ansprüche 1 bis 9, **dadurch gekennzeichnet, daß** der gereinigte vWF in einem Medium mit physiologischer Ionenstärke bzw. physiologischer Osmolalität gewonnen wird und ohne weitere Reinigungsschritte zu einem pharmazeutischen Präparat verarbeitet wird.

12. Verfahren nach einem der Ansprüche 1 bis 11, **dadurch gekennzeichnet, daß** das Kollagen ein Kollagen Typ I oder ein Kollagen Typ III ist.

13. Verfahren nach einem der Ansprüche 1 bis 12, **dadurch gekennzeichnet, daß** ein Kollagen humanen Ursprungs oder vom Schwein, Rind, Ratte oder Pferd verwendet wird.

14. Verfahren nach einem der Ansprüche 1 bis 13, **dadurch gekennzeichnet, daß** das Kollagen mindestens eine Modifikation aufweist, durch welche eine stabile Immobilisierung an den festen Träger ausgebildet wird.

15. Verfahren nach Anspruch 14, **dadurch gekennzeichnet, daß** die Modifikation im Kollagen die Ausbildung von reaktiven Aldehydgruppen ist.

16. Verfahren nach einem der Ansprüche 1 bis 13, **dadurch gekennzeichnet, daß** als Träger ein Chromatographiematerial verwendet wird, an den das Kollagen vorzugsweise kovalent gebunden ist.

17. Verfahren nach einem der Ansprüche 1 bis 13 und 16, **dadurch gekennzeichnet, daß** der Träger prozessiertes Kollagen umfaßt.

18. Verfahren nach einem der Ansprüche 1 bis 13, 16 und 17, **dadurch gekennzeichnet, daß** der vWF und/oder das Kollagen zur Inaktivierung und/oder Abreicherung von Pathogenen, insbesondere von Viren, behandelt ist.

19. Verfahren nach Anspruch 14 oder 15, **dadurch gekennzeichnet, daß** das Eluieren des vWF mit einem Puffer mit einem pH-Wert im Bereich von 5 bis 9 durchgeführt wird.

20. Verfahren nach Anspruch 19, **dadurch gekennzeichnet, daß** das Eluieren des vWF mit einem Puffer mit einem pH-Wert im Bereich von 3 bis 4 durchgeführt wird.

21. Verfahren nach einem der Ansprüche 14, 15, 19 oder 20, **dadurch gekennzeichnet, daß** das Eluieren des vWF mit einem Puffer, enthaltend ein chaotropes Salz, vorzugsweise Ammonium- und/oder Kaliumthiocyanat, durchgeführt wird, wobei die Konzentration an chaotropem Salz vorzugsweise 1 bis 3 M beträgt.

22. Präparat zur Herstellung eines gereinigten vWF umfassend biologisch aktiven vWF, der an immobilisiertes Kollagen gebunden ist.

23. Präparat nach Anspruch 22, **dadurch gekennzeichnet, daß** der vWF eine primäre hämostatische Aktivität ausgedrückt durch eine Kollagenbindungsaktivität von wenigstens 0,9 E vWF:CB/E vWF:Ag, vorzugsweise mindestens 1 E vWF:CB/E vWF:Ag, aufweist und eine Reinheit von wenigstens 70 E vWF:Ag/mg Protein, vorzugsweise mindestens 80 E vWF:Ag/mg Protein.

24. Präparat nach Anspruch 22 oder 23, **dadurch gekennzeichnet, daß** der vWF eine primäre hämostatische Aktivität ausgedrückt in E vWF:CB/E vWF:Ag von wenigstens 0,3, vorzugsweise zumindest 0,4, insbesondere von mindestens 0,6 aufweist.

25. Präparat nach einem der Ansprüche 22 bis 24, **dadurch gekennzeichnet, daß** der vWF als Faktor VIII-vWF-Komplex vorliegt.

26. Präparat nach einem der Ansprüche 22 bis 25, **dadurch gekennzeichnet, daß** der vWF bzw. Faktor VIII-vWF-Komplex frei von plasmatischen Proteinen ist.

27. Präparat nach einem der Ansprüche 22 bis 26, **dadurch gekennzeichnet, daß** das Kollagen an einem festen Träger ausgewählt aus der Gruppe Kollagenpartikel, synthetisches Trägermaterial, Material auf Kohlehydratbasis, Phospholipid und Liposom immobilisiert ist.

28. Präparat nach einem der Ansprüche 22 bis 27, **dadurch gekennzeichnet, daß** das Kollagen natives Kollagen oder ein Kollagenderivat, insbesondere ein enzymatisch prozessiertes Kolla gen oder ein Kollagenfragment, ist.

29. Präparat nach einem der Ansprüche 22 bis 28, **dadurch gekennzeichnet, daß** sie in lyophilisierter Form vorliegt.

30. Präparat nach einem der Ansprüche 22 bis 29, **dadurch gekennzeichnet, daß** sie zur Inaktivierung und/oder Abreicherung gegebenenfalls vorhandener Pathogene behandelt ist.

## Claims

1. A method of chromatographically purifying or fractionating, respectively, von Willebrand factor (vWF) from a vWF-containing starting material, comprising the following steps:
- adsorbing the vWF from the starting material on collagen immobilized on a carrier, with the exception of gelatine sepharose, the collagen being selected from native collagen, a collagen derivative, in particular enzymatically processed collagen, or a collagen fragment, without the binding property of the collagen relative to vWF being adversely affected,
- separating the non-adsorbed portion and, optionally, washing the carrier,
- eluting the vWF from immobilized collagen, and
- recovering the purified vWF.

2. A method according to claim 1, **characterized in that** the vWF is recovered in more than one fraction.

3. A method according to claim 1 or 2, **characterized in that** the vWF is recovered in a first fraction and at least in a second fraction, the elution of the second fraction being carried out with a solution whose ionic strength is increased by at least 20% as compared to the elution solution for the first fraction.

4. A method according to any one of claims 1 to 3, **characterized in that** elution is carried out as a gradient elution.

5. A method according to any one of claims 1 to 4, **characterized in that** a first fraction is obtained in which vWF has a collagen binding activity of less than 0.4 U vWF:CB/U vWF:Ag, and a second fraction, respectively, in which vWF has a collagen binding activity of more than 0.6 U vWF:CB/U vWF:Ag.

6. A method according to any one of claims 1 to 5, **characterized in that** the elution of the second fraction is carried out with a buffer having an ionic strength corresponding to an NaCl concentration of at least 150 mmol/l, in particular corresponding to an NaCl concentration in the range of from 150 to 400 m-mol/l.

7. A method according to any one of claims 1 to 6, **characterized in that** the elution of the first fraction is carried out with a buffer having an ionic strength corresponding to an NaCl concentration of less than 200 mmol/l, in particular corresponding to an NaCl concentration in the range of from 50 to 150 mmol/l, preferably in a medium having physiological ionic strength.

8. A method according to any one of claims 1 to 7, **characterized in that** fibronectin is recovered in a further fraction.

9. A method according to any one of claims 1 to 8, **characterized in that** the vWF-containing starting material is a vWF concentrate or a factor VIII/vWF-complex containing preparation.

10. A method according to any one of claims 1 to 9, **characterized in that** before the adsorption step on immobilized collagen, a pre-purification of the vWF-containing starting material, in particular an ion exchange chromatography, a gel permeation chromatography and/or a precipitation, preferably a precipitation with glycine or ammonium sulfate, are carried out.

11. A method according to any one of claims 1 to 9, **characterized in that** the purified vWF is recovered in a medium with physiological ionic strength and physiological osmolality, respectively, and is processed to a pharmaceutical preparation without further purification steps.

12. A method according to any one of claims 1 to 11, **characterized in that** the collagen is a collagen type I or a collagen type III.

13. A method according to any one of claims 1 to 12, **characterized in that** a collagen of human origin or porcine or bovine collagen or collagen from rat or equine collagen is used.

14. A method according to any one of claims 1 to 13, **characterized in that** the collagen has at least one modification, by which a stable immobilisation to the solid carrier is formed.

15. A method according to claim 14, **characterized in that** the modification in the collagen is the formation of reactive aldehyde groups.

16. A method according to any one of claims 1 to 13, **characterized in that** as carrier a chromatographic material is used to which the collagen is, preferably covalently, bound.

17. A method according to any one of claims 1 to 13 and 16, **characterized in that** the carrier comprises processed collagen.

18. A method according to any one of claims 1 to 13, 16 and 17, **characterized in that** the vWP and/or the collagen is treated for inactivation and/or depletion of pathogens, in particular of viruses.

19. A method according to claim 14 or 15, **characterized in that** eluting of vWF is carried out with a buffer having a pH in the range of from 5 to 9.

20. A method according to claim 19, **characterized in that** eluting of vWF is carried out with a buffer having a pH in the range of from 3 to 4.

21. A method according to any one of claims 14, 15, 19 or 20, **characterized in that** elution of the vWF is carried out with a buffer containing a chaotropic salt, preferably ammonium and/or potassium thiocyanate, the concentration of chaotropic salt preferably being 1 to 3 M.

22. A preparation for producing a purified vWF, comprising biologically active vWF which is bound to immobilized collagen.

23. A preparation according to claim 22, **characterized in that** the vWF has a primary hemostatic activity expressed by a collagen binding activity of at least 0.9 U vWF:CB/U vWF:Ag, preferably at least 1 U vWF:CB/U vWF:Ag, and a purity of at least 70 U vWF:Ag/mg protein, preferably at least 80 U vWF:Ag/mg protein.

24. A preparation according to claim 22 or 23, **characterized in that** the vWF has a primary hemostatic activity expressed in U vWF:CB/U vWF:Ag of at least 0.3, preferably at least 0.4, in particular of at least 0.6.

25. A preparation according to any one of claims 22 to 24, **characterized in that** the vWF is present as a factor VIII/vWF-complex.

26. A preparation according to any one of claims 22 to 25, **characterized in that** the vWF or the factor VIII/vWF-complex, respectively, is free from plasmatic proteins.

27. A preparation according to any one of claims 22 to 26, **characterized in that** the collagen is immobilized on a solid carrier selected from the group of collagen particles, synthetic carrier material, carbohydrate-based material, phospholipid and liposome.

28. A preparation according to any one of claims 22 to 27, **characterized in that** the collagen is native collagen or a collagen derivative, in particular an enzymatically processed collagen or a collagen fragment.

29. A preparation according to any one of claims 22 to 28, **characterized in that** it is present in lyophilized form.

30. A preparation according to any one of claims 22 to 29, **characterized in that** it is treated for inactivation and/or depletion of pathogens possibly present.

## Revendications

1. Procédé de purification chromatographique et respectivement, de fractionnement du facteur de von Willebrand (vWF) à partir d'un matériau de départ contenant le vWF, comprenant les étapes consistant à :
- adsorber le vWF à partir du matériau de départ sur du collagène immobilisé sur un support, à l'exception de la gélatine-sépharose, où le collagène est choisi parmi le collagène natif, un dérivé du collagène, en particulier un collagène traité de manière enzymatique, ou un fragment de collagène, où la propriété de liaison du collagène au vWF n'a pas été influencée défavorablement ;
- séparer la partie non adsorbée et le cas échéant, laver le support ;
- éluer le vWF du collagène immobilisé, et
- récupérer le vWF purifié.

2. Procédé selon la revendication 1, **caractérisé en ce que** le vWF est obtenu en plus d'une fraction.

3. Procédé selon la revendication 1 ou 2, **caractérisé en ce que** le vWF est obtenu dans une première fraction et au moins une deuxième fraction, où l'élution de la deuxième fraction est réalisée avec une solution qui présente une force ionique augmentée d'au moins 20% par rapport à la solution d'élution de la première fraction.

4. Procédé selon l'une quelconque des revendications 1 à 3, **caractérisé en ce que** l'élution est réalisée sous la forme d'un gradient d'élution.

5. Procédé selon l'une quelconque des revendications 1 à 4, **caractérisé en ce que** l'on obtient une première fraction, dans laquelle le vWF présente une activité de liaison au collagène de moins de 0,4 E vwF:CB/E vWF:Ag et respectivement, une deuxième fraction, dans laquelle le vWF présente une activité de liaison au collagène de plus de 0,6 E vwF:CB/E vWF:Ag.

6. Procédé selon l'une quelconque des revendications 1 à 5, **caractérisé en ce que** l'élution de la deuxième fraction est effectuée avec un tampon ayant une force ionique correspondant à une concentration en NaCl d'au moins 150 mmoles/litre, en particulier correspondant à une concentration en NaCl située dans l'intervalle allant de 150 à 400 mmoles/litre.

7. Procédé selon l'une quelconque des revendications 1 à 6, **caractérisé en ce que** l'élution de la première fraction est effectuée avec un tampon ayant une force ionique correspondant à une concentration en NaCl inférieure à 200 mmoles/litre, en particulier correspondant à une concentration en NaCl située dans l'intervalle allant de 50 à 150 mmoles/litre, de préférence dans un milieu avec une force ionique physiologique.

8. Procédé selon l'une quelconque des revendications 1 à 7, **caractérisé en ce que** l'on obtient la fibronectine dans une autre fraction.

9. Procédé selon l'une quelconque des revendications 1 à 8, **caractérisé en ce que** le matériau de départ contenant le vWF est un concentré de vWF ou une préparation contenant le complexe facteur VIII-vWF.

10. Procédé selon l'une quelconque des revendications 1 à 9, **caractérisé en ce que**, avant l'étape d'adsorption sur le collagène immobilisé, on réalise une prépurification du matériau de départ contenant le vWF, en particulier une chromatographie échangeuse d'ions, une chromatographie de perméation sur gel et/ou une précipitation, de préférence une précipitation avec la glycine ou le sulfate d'ammonium.

11. Procédé selon l'une quelconque des revendications 1 à 9, **caractérisé en ce que** l'on obtient le vWF purifié dans un milieu ayant une force ionique physiologique ou une osmolalité physiologique et on traite sans autre étape de purification en une préparation pharmaceutique.

12. Procédé selon l'une quelconque des revendications 1 à 11, **caractérisé en ce que** le coliagène est un collagène de type I ou un collagène de type III.

13. Procédé selon l'une quelconque des revendications 1 à 12, **caractérisé en ce que** l'on utilise un collagène d'origine humaine ou de porc, de bovin, de rat ou de cheval.

14. Procédé selon l'une quelconque des revendications 1 à 13, **caractérisé en ce que** le collagène présente au moins une modification par laquelle une immobilisation stable est formée sur le support solide.

15. Procédé selon la revendication 14, **caractérisé en ce que** la modification dans le collagène est la formation de radicaux aldéhyde réactifs.

16. Procédé selon l'une quelconque des revendications 1 à 13, **caractérisé en ce que** l'on utilise comme support, un matériau de chromatographie, sur lequel le collagène est de préférence, lié de manière covalente.

17. Procédé selon l'une quelconque des revendications 1 à 13 et 16, **caractérisé en ce que** le support comprend le collagène traité.

18. Procédé selon l'une quelconque des revendications 1 à 13, 16 et 17, **caractérisé en ce que** le vWF et/ou le collagène est traité pour l'inactivation et/ou l'appauvrissement des pathogènes, en particulier des virus.

19. Procédé selon la revendication 14 ou 15, **caractérisé en ce que** l'élution du vWF est réalisée avec un tampon ayant un pH situé dans l'intervalle allant de 5 à 9.

20. Procédé selon la revendication 19, **caractérisé en ce que** l'élution du vWF est réalisée avec un tampon ayant un pH situé dans l'intervalle allant de 3 à 4.

21. Procédé selon l'une quelconque des revendications 14, 15, 19 ou 20, **caractérisé en ce que** l'élution du vWF est réalisée avec un tampon contenant un sel chaotrope, de préférence le thiocyanate d'ammonium et/ou de potassium, où la concentration du sel chaotrope se situe de préférence dans l'intervalle allant de 1 à 3 M.

22. Composition pour la préparation d'un vWF purifié comprenant un vWF biologiquement actif, qui est lié au collagène immobilisé.

23. Composition selon la revendication 22, **caractérisée en ce que** le vWF présente une activité hémostatique primaire exprimée par une activité de liaison au collagène d'au moins 0,9 E vwF:CB/E vWF:Ag, de préférence au moins 1 E vwF:CB/E vWF:Ag et une pureté d'au moins 70 E vwF:Ag/mg de protéine, de préférence au moins 80 E vwF:Ag/mg de protéine.

24. Composition selon la revendication 22 ou 23, **caractérisée en ce que** le vWF présente une activité hémostatique primaire exprimée en E vwF:CB/E vWF:Ag d'au moins 0,3, de préférence au moins 0,4, en particulier au moins 0,6.

25. Composition selon l'une quelconque des revendications 22 à 24, **caractérisée en ce que** le vWF est présent sous la forme du complexe facteur VIII-vWF.

26. Composition selon l'une quelconque des revendications 22 à 25, **caractérisée en ce que** le vWF ou le complexe Facteur VIII-vWF est exempt de protéine plasmatique.

27. Composition selon l'une quelconque des revendications 22 à 26, **caractérisée en ce que** le collagène est immobilisé sur un support solide choisi parmi le groupe des particules de collagène, des matériaux supports synthétiques, d'un matériau à base d'hydrate de carbone, des phospholipides et des liposomes.

28. Composition selon l'une quelconque des revendications 22 à 27, **caractérisée en ce que** le collagène est choisi parmi le collagène natif ou un dérivé du collagène, en particulier un collagène traité de manière enzymatique, ou un fragment de collagène.

29. Composition selon l'une quelconque des revendications 22 à 28, **caractérisée en ce qu'**elle est sous forme lyophilisée.

30. Composition selon l'une quelconque des revendications 22 à 29, **caractérisée en ce qu'**elle est traitée pour l'inactivation et/ou l'appauvrissement en les pathogènes le cas échéant présents.
